# EUROPEAN PATENT APPLICATION

(11) **EP 2 057 997 A1**
(43) Date of publication of application: **13.05.2009**
(21) Application number: 07792845.5
(22) Date of filing: 22.08.2007
(51) Int. Cl.: A61K 38/22, A61K 31/337, A61K 45/00, A61P 25/02, A61P 35/00, A61P 43/00

(54) **PREVENTIVE AND/OR THERAPEUTIC AGENT FOR PERIPHERAL NEUROPATHY**

(30) Priority: 22.08.2006 JP 2006225691
(71) Applicant: CHUGAI SEIYAKU KABUSHIKI KAISHA, Tokyo, 115-8543 (JP)
(72) Inventor: SUGAWA, Makoto, Tokyo 103-8324 (JP)
(74) Representative: Harmsen, Dirk
(86) International application number: PCT/JP2007/066246
(87) International publication number: WO 2008/023725

(57) **Abstract**

When a pharmaceutical composition for preventing and/or treating peripheral neuropathy caused by chromosomal microtubule inhibition, which comprises erythropoietin as an active ingredient, is administered to patients receiving a microtubule inhibitor as an anticancer agent, such treatment alleviates peripheral neuropathy in the patients and enables increased dosage, prolonged period and increased frequency for administration of the above anticancer agent, which were never before achieved. Thus, the pharmaceutical composition not only contributes to improvement of QOL in the patients, but also enables prolongation of life in the patients.

## Description

### TECHNICAL FIELD

The present invention relates to a novel pharmaceutical composition for preventing and/or treating peripheral neuropathy caused by microtubule inhibition, which comprises erythropoietin as an active ingredient.

### BACKGROUND ART

Cisplatin and derivatives thereof, as well as taxan-based anticancer agents have excellent therapeutic effects and are widely used in clinical practice. However, these agents also cause serious side effects, i.e., anemia and peripheral neuropathy. Peripheral neuropathy is a disease with symptoms of pain, which causes degeneration of nerve axons and/or myelin disruption. It is well known that peripheral neuropathy is not only caused by treatment with anticancer agents, but also occurs as a complication of HIV infection or diabetes.

On the other hand, erythropoietin (EPO) has been regarded as a hematopoietic cytokine. Recent reports have indicated that EPO is also produced in the central nervous system (Masuda et al., 1994), is involved in the development of the nervous system (Liu et al., 1994; Juul et al., 1998; 1999; Chin et al., 2000) and shows a wide neuroprotective effect in diseases such as brain ischemia (Sakanaka et al., 1998; Briness et al., 2000), spinal cord injury (Gorio et al., 2002), encephalitis (Agnello et al., 2002), etc.

Moreover, the presence of EPO and EPO receptors in the peripheral nervous system has also been reported in rats (Campana & Myers, 2001) and human tissue (Hassan et al., 2004). This suggests that EPO also plays a physiological role in the peripheral nervous system.

As supporting evidence for physiological functions of EPO in the peripheral nervous system, the group of Campana et al. has reported that when sciatic nerves of rats are injured or crushed by chronic compression, EPO expression is increased in glia cells (Schwann cells) near the injury site (Li et al., 2005). When injured, the sciatic nerves undergo Wallerian degeneration (axonal degeneration) on the distal side, while the nerve segment proximal to the injury site serves as a locus for neurite sprouting (Ide, 1996; Cheng & Zochodne, 2002). As a role of Schwann cells in peripheral neuropathy, it is known that Schwann cells are redifferentiated and/or proliferated, and further migrate in the injury site and its distal side, thereby playing an essential role as a scaffold for sprouting of new neurites or regenerated axons (Hall & Gregson, 1974; Ide, 1996; Li et al., 2005). Schwann cells are glia cells responsible for myelin formation in peripheral nerves and hence correspond to oligodendrocytes in the central nervous system. The inventors of the present invention have reported that EPO is a molecule involved in oligodendrocyte maturation and myelination, and may stimulate remyelination in demyelinating disease of the central nervous system (Sugawa et al., 2002; International Patent Publication No. WO02/02135 A1). These findings suggest that EPO acts not only on nerve cells in the central nervous system, but also on Schwann cells, and may be involved in nerve protection and/or nerve regeneration and repair during peripheral neuropathy.

As to the effects of exogenous EPO on neuropathy, there are reports showing that EPO prevents cell loss in DRG (dorsal root ganglia) caused by addition of HIV envelop glycoprotein gp120, which is a model of HIV-associated sensory neuropathy (HIV-SN), a troublesome neurological symptom most commonly caused by HIV infection (Keswanai et al., 2004), and showing that EPO improves reduced nerve conduction velocity and pain in streptozotocin-induced diabetes model rats (Bianchi et al., 2004).

Moreover, it has been clinically reported that the neurological symptom scores of cisplatin-treated cancer patients were significantly improved in the EPO-treated group (Mangiameli et al., 2002).

However, cisplatin-induced peripheral neuropathy is due to radical generation, and it is completely unknown whether EPO has a prophylactic or therapeutic effect on other types of peripheral neuropathy with different onset mechanisms. In particular, there is no report about the effects of EPO on peripheral neuropathy induced by a microtubule inhibitor, when administered as an anticancer agent, which kills cancer cells by stopping the action of chromosomal microtubules having an important contribution to cell division.
Non-patent Document 1: Agnello D, Bigini P, Villa P, Mennini T, Cerami A, Brines ML, Ghezzi P. Erythropoietin exerts an anti-inflammatory effect on the CNS in a model of experimental autoimmune encephalomyelitis,. Brain Res. 2002 Oct 11;952(1):128-34.
Non-patent Document 2: Bianchi R, Buyukakilli B, Brines M, Savino C, Cavaletti G, Oggioni N, Lauria G, Borgna M, Lombardi R, Cimen B, Comelekoglu U, Kanik A, Tataroglu C, Cerami A, Ghezzi P. Erythropoietin both protects from and reverses experimental diabetic neuropathy,. Proc Natl Acad Sci U S A. 2004 Jan 20;101(3):823-8.
Non-patent Document 3: Brines ML, Ghezzi P, Keenan S, Agnello D, de Lanerolle NC, Cerami C, Itri LM, Cerami A. Erythropoietin crosses the blood-brain barrier to protect against experimental brain injury,. Proc Natl Acad Sci U S A. 2000 Sep 12;97(19):10526-31.
Non-patent Document 4: Campana WM, Myers RR. Erythropoietin and erythropoietin receptors in the peripheral nervous system: changes after nerve injury, FASEB J. 2001 Aug;15(10):1804-6.
Non-patent Document 5: Campana WM, Myers RR. Exogenous erythropoietin protects against dorsal root ganglion apoptosis and pain following peripheral nerve injury,. Eur J Neurosci. 2003 Sep;18(6):1497-506.
Non-patent Document 6: Cheng C, Zochodne DW. In vivo proliferation, migration and phenotypic changes of Schwann cells in the presence of myelinated fibers. Neuroscience. 2002;115(1):321-9.
Non-patent Document 7: Gorio A, Gokmen N, Erbayraktar S, Yilmaz O, Madaschi L, Cichetti C, Di Giulio AM, Vardar E, Cerami A, Brines M. Recombinant human erythropoietin counteracts secondary injury and markedly enhances neurological recovery from experimental spinal cord trauma. Proc Natl Acad Sci U S A. 2002 Jul 9;99(14):9450-5.
Non-patent Document 8: Hall SM, Gregson NA. The effects of mitomycin C on remyelination in the peripheral nervous system. Nature. 1974 Nov 22;252(5481):303-5.
Non-patent Document 9: Hassan K, Simri W, Rubenchik I, Manelis J, Gross B, Shasha SM, Kristal B. Effect of erythropoietin therapy on polyneuropathy in predialytic patients. J Nephrol. 2003 Jan-Feb;16(1):121-5.
Non-patent Document 10: Hassan K, Gross B, Simri W, Rubinchik I, Cohen H, Jacobi J, Shasha SM, Kristal B. The presence of erythropoietin receptors in the human peripheral nervous system. Clin Nephrol. 2004 Feb;61(2):127-9.
Non-patent Document 11: Ide C. Peripheral nerve regeneration. Neurosci Res. 1996 Jun;25(2):101-21. Review.
Non-patent Document 12: Juul SE, Yachnis AT, Rojiani AM, Christensen RD. Immunohistochemical localization of erythropoietin and its receptor in the developing human brain. Pediatr Dev Pathol. 1999 Mar-Apr;2(2):148-58.
Non-patent Document 13: Juul SE, Anderson DK, Li Y, Christensen RD. Erythropoietin and erythropoietin receptor in the developing human central nervous system. Pediatr Res. 1998 Jan;43(1):40-9.
Non-patent Document 14: Keswani SC, Leitz GJ, Hoke A. Erythropoietin is neuroprotective in models of HIV sensory neuropathy. Neurosci Lett. 2004 Nov 23;371(2-3):102-5.
Non-patent Document 15: Li X, Gonias SL, Campana WM. Schwann cells express erythropoietin receptor and represent a major target for Epo in peripheral nerve injury. Glia. 2005 Apr 5
Non-patent Document 16: Liu ZY, Chin K, Noguchi CT. A minimal cytoplasmic subdomain of the erythropoietin receptor mediates erythroid and megakaryocytic cell development. Blood. 1999 Nov 15;94(10):3381-7.
Non-patent Document 17: Mangiameli A, Spina S, Iannetti E, Catalano D, Spadaro D, Trovato GM. Erythropoietin and cisplatin-induced neuropathies in cancer patients. Clin Ter. 2002 May-Jun;153(3):177-80. Italian.
Non-patent Document 18: Masuda S., Okano M., Yamagishi K., Nagao M., Ueda M., Sasaki R. A novel site of erythropoietin production: oxygen-dependent production in cultured rat astrocytes., J Biol. Chem. 269, 19488-19493.
Non-patent Document 19: Sakanaka M, Wen TC, Matsuda S, Masuda S, Morishita E, Nagao M, Sasaki R. In vivo evidence that erythropoietin protects neurons from ischemic damage. Proc Natl Acad Sci U S A. 1998 Apr 14;95(8):4635-40.
Non-patent Document 20: Sugawa M, Sakurai Y, Ishikawa-Ieda Y, Suzuki H, Asou H. Effects of erythropoietin on glial cell development; oligodendrocyte maturation and astrocyte proliferation. Neurosci Res. 2002 Dec;44(4):391-403.

### DISCLOSURE OF THE INVENTION

### PROBLEMS TO BE SOLVED BY THE INVENTION

The object of the present invention is to provide a pharmaceutical composition for preventing and/or treating peripheral neuropathy, more particularly a pharmaceutical composition for preventing and/or treating peripheral neuropathy caused by microtubule inhibition.

### MEANS FOR SOLVING THE PROBLEMS

The inventors of the present invention have found that erythropoietin has a significant therapeutic effect on peripheral neuropathy caused by a microtubule inhibitor (e.g., taxotere) when administered as an anticancer agent. This finding led to the completion of the present invention.

Namely, the present invention provides the following.
(1) A pharmaceutical composition for preventing and/or treating peripheral neuropathy caused by microtubule inhibition, which comprises erythropoietin as an active ingredient.
(2) The pharmaceutical composition according to (1) above, wherein the peripheral neuropathy caused by microtubule inhibition is that caused by administration of at least one member selected from vinca alkaloids or taxans.
(3) The pharmaceutical composition according to (2) above, wherein a member of the taxans is paclitaxel or taxotere.
(4) Use of an erythropoietin-containing pharmaceutical composition for the purpose of cancer therapy, wherein the pharmaceutical composition is used in combination with an anticancer agent having an inhibitory effect on microtubules.
(5) A method for preventing or treating peripheral neuropathy caused by microtubule inhibition, which comprises administering a pharmaceutical composition containing a prophylactically or therapeutically effective amount of erythropoietin to a patient in need of such prevention or treatment.
(6) A method for cancer therapy, which comprises administering to a patient a pharmaceutical composition containing erythropoietin in an amount effective for preventing or treating peripheral neuropathy caused by microtubule inhibition, wherein the pharmaceutical composition is administered in combination with an anticancer agent having an inhibitory effect on microtubules.
(7) Use of erythropoietin in the manufacture of a pharmaceutical composition for preventing or treating peripheral neuropathy caused by microtubule inhibition.
(8) A combined formulation, which comprises a pharmaceutical composition containing erythropoietin in an amount effective for preventing or treating peripheral neuropathy caused by microtubule inhibition and an anticancer agent having an inhibitory effect on microtubules.

### ADVANTAGES OF THE INVENTION

As shown in the Example section described later, in taxotere-induced peripheral neuropathy model mice, EPO was observed to alleviate peripheral neuropathy both in evaluation of neurological symptoms and in histopathological evaluation of sciatic nerves.

Thus, the prophylactic and/or therapeutic agents of the present invention are useful for peripheral neuropathy and particularly useful for reducing side effects of peripheral neuropathy in patients receiving a microtubule inhibitor as an anticancer agent. As a result, the agents of the present invention not only alleviate pain in the patients (improves their QOL), but also enable increased dosage, prolonged period and increased frequency for administration of the anticancer agent, which were never before achieved. The agents also enable prolongation of life.

### BRIEF DESCRIPTION OF THE DRAWINGS

[Figure 1] Figure 1 is a graph showing neurological symptom changes induced by taxotere treatment.
[Figure 2] Figure 2 is a graph showing neurological symptom changes induced by taxotere treatment at 90 mg/kg ip and the effect of EPO administration thereon.
[Figure 3-1] Figure 3-1 presents photographs showing histopathological changes in degeneration of sciatic nerve axons and the effect of EPO administration thereon in the group not receiving taxotere (0 iv), as analyzed by toluidine blue staining.
[Figure 3-2] Figure 3-2 presents photographs showing histopathological changes in degeneration of sciatic nerve axons and the effect of EPO administration thereon in the group receiving taxotere (90 mg/kg ip), as analyzed by toluidine blue staining.

### BEST MODE FOR CARRYING OUT THE INVENTION

### Erythropoietin

The erythropoietin used in the present invention can be any EPO, but preferably highly purified EPO, more specifically mammalian EPO, especially having biological activity substantially identical to that of human EPO.

The erythropoietin used in the present invention can be those prepared by any process, including, e.g., natural human EPO purified from human-derived extracts (JPB HEI 1-38800, etc.), or human EPO recombinantly produced in E. coli, yeast cells, Chinese hamster ovary cells (CHO cells), C127 cells, COS cells, myeloma cells, BHK cells, insect cells or the like and extracted and isolated/purified by various methods. The erythropoietin used in the present invention is preferably recombinantly prepared, preferably by using mammalian cells (especially CHO cells) (e.g., JPB HEI 1-44317, Kenneth Jacobs et al., Nature, 313 806-810 (1985), etc.).

Recombinantly obtained EPO may have an amino acid sequence identical to that of naturally derived EPO or may contain a deletion, substitution, addition or other modification of one or more amino acids in the amino acid sequence so far as it has similar biological activity to that of naturally derived EPO. Amino acid deletion, substitution, addition or other modification can be performed by methods known to those skilled in the art. For example, a polypeptide functionally comparable to EPO can be prepared by those skilled in the art by introducing an amino acid variation into EPO as appropriate via site-directed mutagenesis (Gotoh, T. et al. (1995) Gene 152, 271-275; Zoller, M.J. and Smith, M. (1983) Methods Enzymol. 100, 468-500; Kramer, W. et al. (1984) Nucleic Acids Res. 12, 9441-9456; Kramer, W. and Fritz, H.J. (1987) Methods Enzymol. 154, 350-367; Kunkel, T.A. (1985) Proc. Natl. Acad. Sci. USA. 82, 488-492; Kunkel (1988) Methods Enzymol. 85, 2763-2766) or other techniques. Amino acid variations also occur in nature. Generally, an amino acid residue is preferably substituted by another amino acid residue in which the property of the amino acid side chain is conserved. For example, the properties of amino acid side chains include hydrophobic amino acids (A, I, L, M, F, P, W, Y, V), hydrophilic amino acids (R, D, N, C, E, Q, G, H, K, S, T), amino acids having aliphatic side chains (G, A, V, L, I, P), amino acids having hydroxyl-containing side chains (S, T, Y), amino acids having sulfur-containing side chains (C, M), amino acids having carboxylate- and amide-containing side chains (D, N, E, Q), amino acids having base-containing side chains (R, K, H), and amino acids having aromatic-containing side chains (H, F, Y, W) (examples shown by one-letter amino acid codes within parentheses). It has been already known that polypeptides having an amino acid sequence modified by deleting, adding and/or substituting one or more amino acid residues retain their biological activity (Mark, D.F. et al., Proc. Natl. Acad. Sci. USA (1984) 81, 5662-5666; Zoller, M.J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413).

For the purpose of the present invention, fusion proteins of EPO and another protein can also be used. Fusion proteins can be prepared by, e.g., ligating the DNA encoding EPO in-frame with the DNA encoding another protein, inserting the ligation product into an expression vector and expressing it in a host. The second protein to be fused to EPO in the present invention is not specifically limited.

For the purpose of the present invention, chemically modified EPO can also be used. Examples of chemically modified EPO include, for example, EPO chemically modified with polyethylene glycol or the like (e.g., WO90/12874), carbohydrate-free EPO chemically modified with polyethylene glycol or the like, as well as EPO conjugated to a compound such as an inorganic or organic compound, e.g., vitamin B12, etc.

Moreover, for the purpose of the present invention, EPO derivatives can also be used. As used herein, EPO derivative refers to EPO containing a modified amino acid in the EPO molecule or to EPO containing a modified carbohydrate chain in the EPO molecule.

Modifications of carbohydrate chains in EPO molecules include addition, substitution, deletion and the like of carbohydrate chains. Preferred carbohydrate modifications in the present invention include deletion of sialic acids in EPO molecules.

Normally, both of EPO produced by recombinant animal cells and EPO derived from urine are obtained as EPO compositions containing various EPO molecules having different carbohydrate structures. The number of sialic acids attached to the EPO molecules in the EPO compositions depends on the specific EPO molecules, but normally 11 to 15 sialic acids are attached to one EPO molecule. Desialylated EPO (asialoEPO) can be prepared by removing these sialic acids. The number of sialic acids removed by desialylation is not specifically limited, and all sialic acids may be removed, or 1, 2, 3, 4, 5, 6, 7, 8, 9, 10, 11, 12, 13 or 14 sialic acids may be removed. Preferred asialoEPO in the present invention has 10 or less, more preferably 5 or less, most preferably 2 or less sialic acids attached to the EPO molecule. It should be noted that the number of sialic acids used in the present invention is the average number in EPO molecules contained in EPO compositions. The average number of sialic acids per molecule can be determined by methods known to those skilled in the art (EP0428267, etc.).

Desialylated EPO (asialoEPO) can be prepared by methods known to those skilled in the art, e.g., by treating EPO with an enzyme such as sialidase. Sialidases are commercially available (JPA 2005-507426, Nobuo Imai et al., Eur. J. Biochem, 194, 457-462 (1990), etc.).
Moreover, the erythropoietin of the present invention may be EPO with a modified carbohydrate chain, including EPO analogs whose carbohydrate chain is modified, as exemplified by NESP (Novel Erythropoietin Stimulating Protein; disclosed in WO85/02610, WO91/05867, WO95/05465, etc.) which has sialic acid attached at the N-terminal end of EPO.

Modifications of amino acids in EPO molecules include carbamylation, biotinylation, amidination, acetylation, guanidinylation, etc., but a preferred amino acid modification in the present invention is carbamylation.

Amino acid residues modified are not limited, including, e.g., lysine, arginine, glutamic acid, tryptophan, etc., but a preferred amino acid modified in the present invention is lysine.

Thus, an especially preferred embodiment of EPO containing a modified amino acid is EPO containing carbamylated lysine (Marcel L et al. Derivatives of erythropoietin that are tissue protective but not erythropoietic. Science, 2004; 305: 239, Fiordaliso E et al. A nonerythropoietic derivative of erythropoietin protects the myocardium from ischemia-reperfusion injury. PNAS, 2005; 102: 2046, etc.). Carbamylations of EPO include carbamylation mediated by reaction with cyanate ions or the like; alkyl carbamylation mediated by reaction with alkyl isocyanates or the like; aryl carbamylation mediated by reaction with aryl isocyanates or the like.

### Prophylactic and/or therapeutic formulations

The prophylactic and/or therapeutic agents of the present invention can contain suspending agents, solubilizers, stabilizers, isotonizing agents, preservatives, adsorption inhibitors, surfactants, diluents, excipients, pH modifiers, soothing agents, buffers, sulfur-containing reducing agents, antioxidants, etc., as appropriate.

Examples of suspending agents include methylcellulose, Polysorbate 80, hydroxyethylcellulose, gum acacia, gum tragacanth powder, sodium carboxymethylcellulose, polyoxyethylene sorbitan monolaurate, etc.

Solubilizers include polyoxyethylene hydrogenated castor oil, Polysorbate 80, nicotinic acid amide, polyoxyethylene sorbitan monolaurate, Macrogols, castor oil fatty acid ethyl esters, etc.

Stabilizers include dextran 40, methylcellulose, gelatin, sodium sulfite, sodium metasulfite, etc.

Certain amino acids can also be included as stabilizers (e.g., JPA HEI 10-182481, etc.). Amino acids added as stabilizers include free amino acids and salts thereof such as sodium salts, potassium salts, hydrochlorides, etc. Amino acids can be added alone or as a combination of two or more. Amino acids added as stabilizers are not specifically limited, but preferred amino acids include leucine, tryptophan, serine, glutamic acid, arginine, histidine and lysine.

Isotonizing agents include, e.g., D-mannitol, sorbitol, etc.

Preservatives include, e.g., methyl paraoxybenzoate, ethyl paraoxybenzoate, sorbic acid, phenol, cresol, chlorocresol, etc.

Adsorption inhibitors include, e.g., human serum albumin, lecithin, dextran, ethylene oxide/propylene oxide copolymers, hydroxypropylcellulose, methylcellulose, polyoxyethylene hydrogenated castor oil, polyethylene glycol, etc.

Typical examples of surfactants include:
nonionic surfactants, e.g., sorbitan fatty acid esters such as sorbitan monocaprylate, sorbitan monolaurate, sorbitan monopalmitate; glycerin fatty acid esters such as glycerin monocaprylate, glycerin monomyristate, glycerin monostearate; polyglycerin fatty acid esters such as decaglyceryl monostearate, decaglyceryl distearate, decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate; polyoxyethylene sorbitol fatty acid esters such as polyoxyethylene sorbitol tetrastearate, polyoxyethylene sorbitol tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, polyoxyethylene polyoxypropylene cetyl ether; polyoxyethylene alkyl phenyl ethers such as polyoxyethylene nonyl phenyl ether; polyoxyethylene hardened castor oils such as polyoxyethylene castor oil, polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; polyoxyethylene fatty acid amides such as polyoxyethylene stearic acid amide, each of which has an HLB of 6-18;
anionic surfactants, e.g., alkyl sulfates having a C10-18 alkyl group such as sodium cetyl sulfate, sodium lauryl sulfate, sodium oleyl sulfate; polyoxyethylene alkyl ether sulfates having an average EO mole number of 2-4 and a C10-18 alkyl group such as sodium polyoxyethylene lauryl sulfate; alkyl sulfosuccinic acid ester salts having a C8-18 alkyl group such as sodium laurylsulfosuccinate; and
natural surfactants, e.g., lecithin; glycerophospholipids; sphingophospholipids such as sphingomyelin; sucrose fatty acid esters of C12-18 fatty acids. Formulations of the present invention can contain one or more of these surfactants in combination. Preferred surfactants are polyoxyethylene sorbitan fatty acid esters such as Polysorbate 20, 40, 60 or 80, especially Polysorbates 20 and 80. Polyoxyethylene polyoxypropylene glycols such as poloxamers (e.g., Pluronic F-68®) are also preferred.

Sulfur-containing reducing agents include, e.g., sulfhydryl-containing compounds such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acid having 1 to 7 carbon atoms.

Antioxidants include, e.g., erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate and sodium metaphosphate.

Other components commonly added may also be contained, e.g., inorganic salts such as sodium chloride, potassium chloride, calcium chloride, sodium phosphate, potassium phosphate and sodium bicarbonate; and organic salts such as sodium citrate, potassium citrate and sodium acetate.

In the prophylactic and/or therapeutic agents of the present invention, the effective dosage of erythropoietin is determined by physicians in consideration of the disease to be treated and its condition, the age and body weight of a patient, the route of administration, etc. In general, EPO is administered at a dose of 0.1 to 5000 µg, preferably 5 to 1000 µg per adult.

Although the prophylactic and/or therapeutic agents of the present invention are generally administered by the parenteral route, for example, in the form of injections (e.g., subcutaneous, intravenous or intramuscular injections) or by the percutaneous, transmucosal, transnasal or transpulmonary route, oral administration is also possible.

### Diseases

The prophylactic and/or therapeutic agents of the present invention are useful for peripheral neuropathy.

Peripheral neuropathy refers to a disorder of the nervous system not including the brain and spinal cord. More specifically, it refers to a peripheral nerve disease that involves disruption of myelin surrounding nerve axons and eventual axonal degeneration. The disorder is observed not only in nerve cells, but also in myelin-forming Schwann cells. Peripheral neuropathy is associated with symptoms such as sensory disturbance, numbness, hypoesthesia, ache, urtication, pain, dysesthesia, anesthesia, dyskinesia, asthenia, paralysis (complete loss of movement), etc.

The prophylactic and/or therapeutic agents of the present invention are particularly useful for peripheral neuropathy which occurs when a microtubule inhibitor is administered as an anticancer agent. A microtubule, which is an intracellular structure, is composed of multiple tubulins (polymerized tubulins). A tubulin dimer and a microtubule are in a "dynamic equilibrium" state through polymerization and depolymerization. An agent which disturbs this equilibrium state and thereby exerts an anticancer effect serves as a microtubule inhibitor. Those known as microtubule inhibitors include: 1) vinca alkaloids (which have an inhibitory effect on microtubule formation through their binding to tubulins to cause binding between tubulins), as exemplified by vincristine, vinblastine, vindesine and vinorelbine; as well as 2) taxans [which stimulate tubulin polymerization to stabilize microtubules, unlike vinca alkaloids. As a result, taxans affect the formation and/or functions of spindles (composed of microtubules) during cell division (M stage) to stop the cell division, thereby causing cell damage, which leads to an anticancer effect] , as exemplified by paclitaxel (taxol), taxotere and docetaxel.

The prophylactic and/or therapeutic agents of the present invention can attain an excellent effect, particularly when used in combination with an anticancer drug, such as paclitaxel or taxotere, which has a strong anticancer effect and often causes neuropathy (e.g., numbness in hands and feet) due to strong inhibition of microtubule action.

The present invention further provides the use of an erythropoietin-containing pharmaceutical composition for the purpose of cancer therapy, wherein the pharmaceutical composition is used in combination with an anticancer agent having an inhibitory effect on microtubules. Such anticancer agents having an inhibitory effect on microtubules are preferably vinca alkaloids and taxans as mentioned above, more preferably taxans. Particularly preferred are paclitaxel and taxotere. The timing at which the erythropoietin-containing pharmaceutical composition of the present invention is combined with the above anticancer agent is determined by physicians in consideration of the effect of erythropoietin as well as the disease to be treated and its condition, the age and body weight of a patient, the route of administration, etc. It may be any timing of before, simultaneously with, or after administration of the above anticancer agent, particularly preferably before or simultaneously with administration of the above anticancer agent.

The present invention further provides a method for preventing or treating peripheral neuropathy caused by microtubule inhibition, which comprises administering a pharmaceutical composition containing a prophylactically or therapeutically effective amount of erythropoietin to a patient in need of such prevention or treatment.

The present invention further provides a method for cancer therapy, which comprises administering to a patient a pharmaceutical composition containing erythropoietin in an amount effective for preventing or treating peripheral neuropathy caused by microtubule inhibition, wherein the pharmaceutical composition is administered in combination with an anticancer agent having an inhibitory effect on microtubules.

The present invention further provides the use of erythropoietin in the manufacture of a pharmaceutical composition for preventing or treating peripheral neuropathy caused by microtubule inhibition.

The present invention further provides a combined formulation, which comprises a pharmaceutical composition containing erythropoietin in an amount effective for preventing or treating peripheral neuropathy caused by microtubule inhibition and an anticancer agent having an inhibitory effect on microtubules.

In the present invention, an embodiment where an erythropoietin-containing pharmaceutical composition is administered "in combination with" an anticancer agent having an inhibitory effect on microtubules covers all cases, i.e., before administration of the anticancer agent, simultaneously with administration of the anticancer agent, and after administration of the anticancer agent.

### EXAMPLES

The following examples further illustrate the present invention without, however, limiting the same thereto. Various changes and modifications can be made by those skilled in the art, and these changes and modifications are also included in the present invention.

### Example 1: Effect of EPO in taxotere-induced peripheral neuropathy model <Experimental Materials and Methods>

### (1) Preparation of taxotere-induced peripheral neuropathy model

### (1-1) Animals

ICR mice (male) were purchased at 5 weeks of age and handled for 1 week before use in the experiment.

### (1-2) Preparation and administration of reagents

As an anticancer agent, taxotere (Taxotere injection, Aventis Pharma, 1V 80 mg 2mL) was used.

Using the attached solution and physiological saline, taxotere was adjusted to a volume of about 0.15 ml per 10 g mouse body weight (45 mg/kg iv, 90 mg/kg ip). The vehicle serving as a control (0 mg/kg) was prepared in the same manner using Tween-80 and physiological saline for iv administration.

On the other hand, recombinant human EPO under the trade name Epogin (Chugai Pharmaceutical Co., Ltd., Japan) was adjusted using a solution (containing 135 nmol/L NaCl, 0.125% histidine-HCl and 0.005% Tween 80 in 10 mmol/L phosphate buffer (pH 6.0)) and administered at 720 U/head/week. The administration schedule was as follows: taxotere was given at 0 or 45 mg/kg iv (via the tail vein) or at 90 mg/kg ip, once a week for 3 weeks (3 times in total on days 0, 7 and 14), while the vehicle and EPO (720 U/head) were each given sc (into the dorsal neck region) 3 hours before taxotere treatment.

The mice were divided into the following 6 groups, with the number of animals given in parentheses.

1. Taxotere 0 iv + Vehicle (5)
2. Taxotere 0 iv + EPO (5)
3. Taxotere 45 iv + Vehicle (8)
4. Taxotere 90 ip + Vehicle (8)
5. Taxotere 90 ip + EPO (8)

### (2) Evaluation of neurological symptoms

Continuous treatment with taxotere will induce typical hind leg crossing in mice when hanged by their tails. If the symptoms reach an advanced stage, the same crossing response will also occur in the forelegs, thereby leading to dysbasia. Such a symptom was used as an indicator to evaluate the mice as follows: 0 = no symptom; 0.5 = abnormal movement without clear flexion; 1 = flexion in one hind leg; 2 = hind leg flexion or extension lasting for 2 seconds or longer; 3 = dysbasia. Scoring was accomplished by blind evaluation of symptoms.

### (3) Histopathological evaluation of sciatic nerves

Muscle tissue was peeled off to expose sciatic nerves. Under this state, a glutaraldehyde fixing solution (stored on ice immediately before use) was added to immerse all the sciatic nerves. After incubation for 10 minutes, the sciatic nerves were isolated and gently shaken for 1 hour in a shaker containing a glutaraldehyde fixing solution. The sciatic nerves were then embedded in a standard manner into a resin for electron microscopic examination. Toluidine blue-stained sections were prepared and evaluated for the degree of axonal degeneration at three levels: none (-), mild (+) and moderate (++).

### <Results>

### (1) Neurological symptom changes

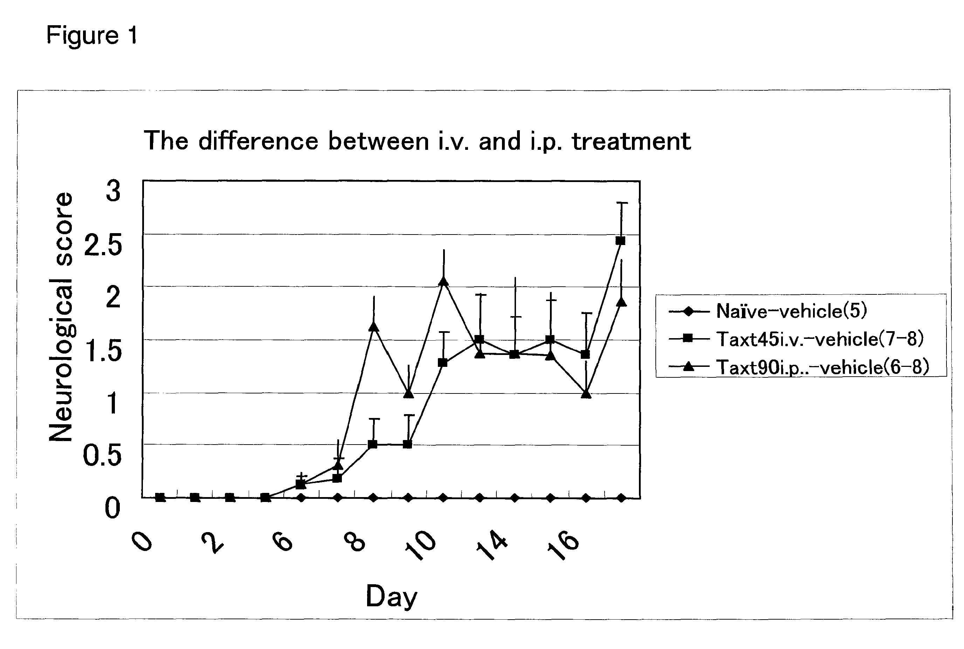

Upon taxotere treatment, neurological symptoms developed around 8 days after the treatment in both cases of 45 mg/kg iv and 90 mg/kg ip. Then, the symptoms became further exacerbated day by day. Although the symptoms tended to be exacerbated faster in the 90 mg/kg ip group than in the 45 mg/kg iv group on days 7 to 10, these groups showed almost the same course of symptoms and no significant difference over the subsequent days (Figure 1). In contrast, the group not receiving taxotere (naive) showed no neurological symptom (Figure 1). Based on these results, taxotere treatment at 90 mg/kg ip was used for examination of drug effects because it allowed faster development of the symptoms.

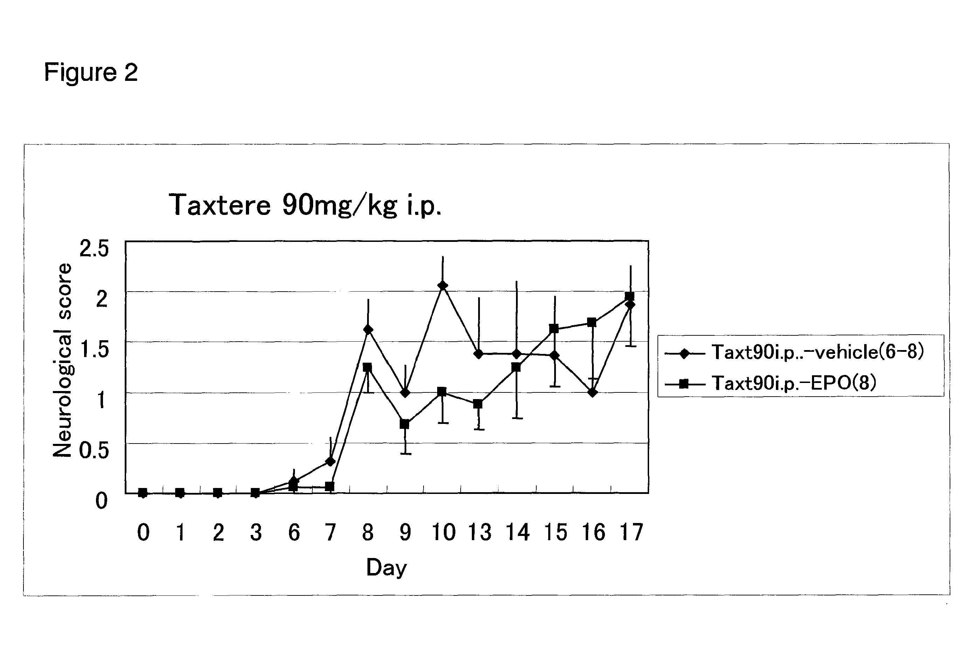

In neuropathy induced by taxotere treatment at 90 mg/kg ip, EPO administration was found to reduce significantly the neurological symptoms after 10 days (Figure 2).

### (2) Histological examination

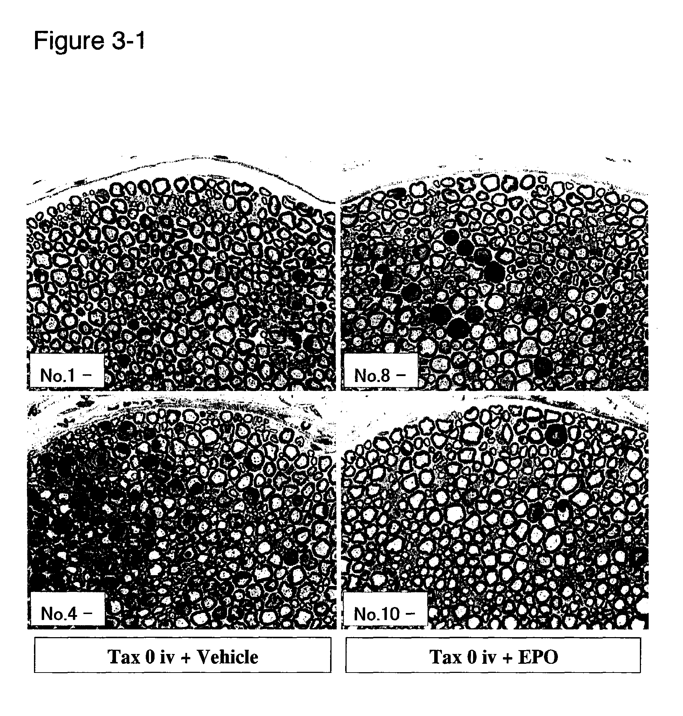

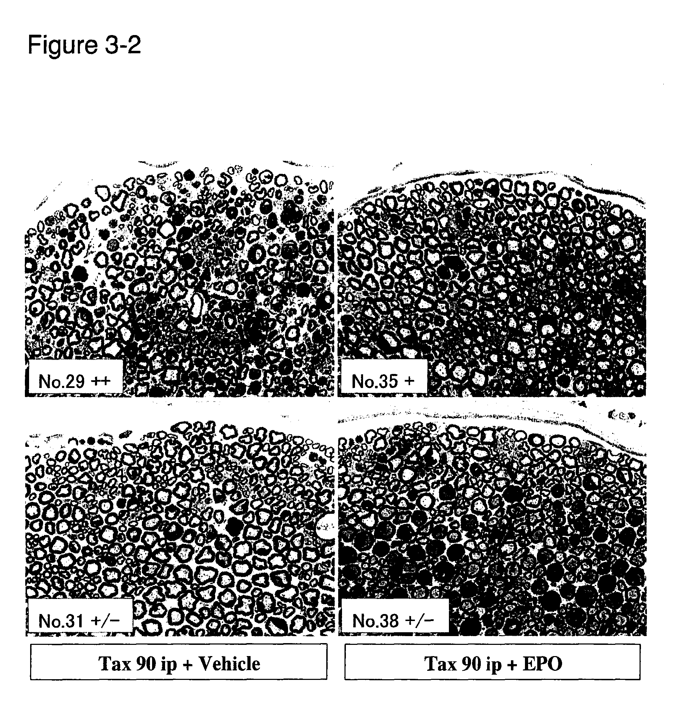

The results of histological examination by toluidine blue staining are shown in Table 1 and Figures 3-1 and 3-2.

[Table 1]

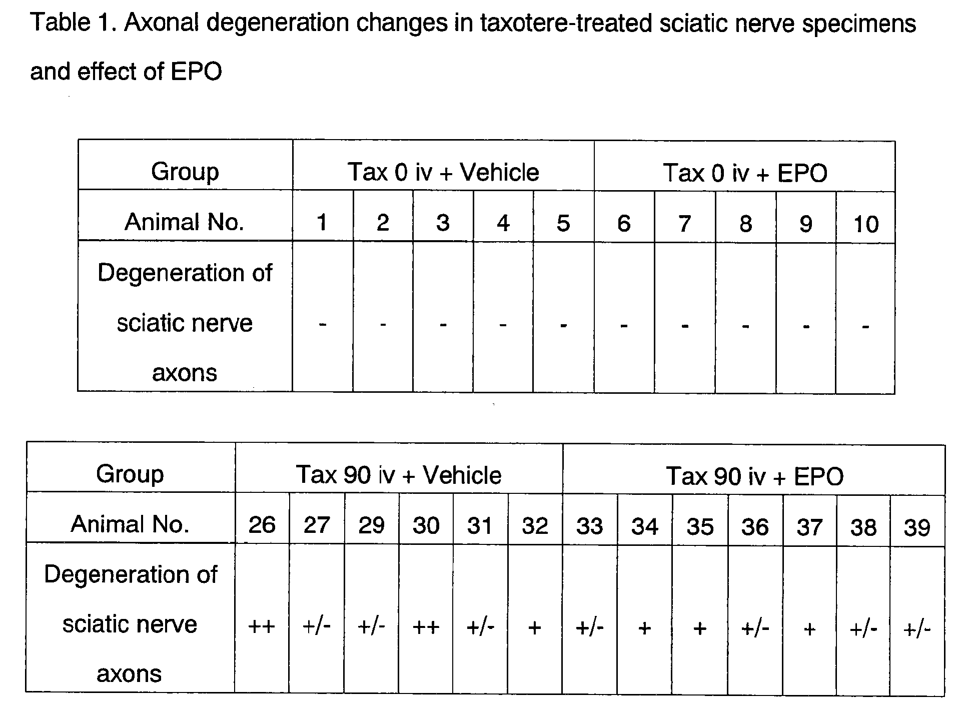

The groups not receiving taxotere (0 iv) showed no degeneration of sciatic nerve axons, whereas the groups receiving taxotere showed diameter reduction and deformation of axons, which were indicative of clear axonal degeneration. The degree of axonal degeneration in the 90 mg/kg ip group was as follows: ±, n = 3; mild (+), n = 1; moderate (++), n = 3 (Figure 3-2, Table 1). In contrast, the group receiving EPO showed the degeneration levels within the range of ± to mild (+) levels even upon 90 mg/kg ip treatment, and the number of animals evaluated as the ± level was larger than in the vehicle group, thus indicating a tendency to alleviate neuropathy (Figure 3-2, Table 1).

These results indicated that EPO administration tended to suppress exacerbation of neurological symptoms and axonal degeneration of sciatic nerves in the peripheral neuropathy model induced by taxotere, an anticancer agent widely used in cancer therapy (Figure 1, Figure 2, Figure 3-1, Figure 3-2, Table 1).

### INDUSTRIAL APPLICABILITY

When a pharmaceutical composition comprising erythropoietin as an active ingredient is administered to patients receiving a microtubule inhibitor as an anticancer agent, such treatment alleviates peripheral neuropathy in the patients and enables increased dosage, prolonged period and increased frequency for administration of the above anticancer agent, which were never before achieved. Thus, the pharmaceutical composition not only contributes to improvement of QOL in the patients, but also enables prolongation of life in the patients.

## Claims

1. A pharmaceutical composition for preventing and/or treating peripheral neuropathy caused by microtubule inhibition, which comprises erythropoietin as an active ingredient.

2. The pharmaceutical composition according to claim 1, wherein the peripheral neuropathy caused by microtubule inhibition is that caused by administration of at least one member selected from vinca alkaloids or taxans.

3. The pharmaceutical composition according to claim 2, wherein a member of the taxans is paclitaxel or taxotere.

4. Use of an erythropoietin-containing pharmaceutical composition for the purpose of cancer therapy, wherein the pharmaceutical composition is used in combination with an anticancer agent having an inhibitory effect on microtubules.

5. A method for preventing or treating peripheral neuropathy caused by microtubule inhibition, which comprises administering a pharmaceutical composition containing a prophylactically or therapeutically effective amount of erythropoietin to a patient in need of such prevention or treatment.

6. A method for cancer therapy, which comprises administering to a patient a pharmaceutical composition containing erythropoietin in an amount effective for preventing or treating peripheral neuropathy caused by microtubule inhibition, wherein the pharmaceutical composition is administered in combination with an anticancer agent having an inhibitory effect on microtubules.

7. Use of erythropoietin in the manufacture of a pharmaceutical composition for preventing or treating peripheral neuropathy caused by microtubule inhibition.

8. A combined formulation, which comprises a pharmaceutical composition containing erythropoietin in an amount effective for preventing or treating peripheral neuropathy caused by microtubule inhibition and an anticancer agent having an inhibitory effect on microtubules.
